(19) Europäisches Patentamt
European Patent Office
Office européen des brevets

(11) Veröffentlichungsnummer: **0 124 630**
**B1**

(12) # EUROPÄISCHE PATENTSCHRIFT

(45) Veröffentlichungstag der Patentschrift: **28.01.87**

(51) Int. Cl.⁴: **C 07 D 401/12,** A 61 K 31/505

(21) Anmeldenummer: **83104452.4**

(22) Anmeldetag: **05.05.83**

(54) **Pyrimidin-Thioalkylpyridin-Derivate, Verfahren zu ihrer Herstellung und diese Verbindung enthaltende Arzneimittel.**

(43) Veröffentlichungstag der Anmeldung:
**14.11.84 Patentblatt 84/46**

(45) Bekanntmachung des Hinweises auf die Patenterteilung:
**28.01.87 Patentblatt 87/5**

(84) Benannte Vertragsstaaten:
**AT BE CH DE FR GB IT LI LU NL SE**

(56) Entgegenhaltungen:
**GB - A - 2 014 560**

**CHEMICAL ABSTRACTS, Band 88, 1978, Seite 774, Nr. 190887g, Columbus, Ohio, USA**

(73) Patentinhaber: **LUDWIG HEUMANN & CO GMBH, Heideloffstrasse 18-28 Postfach 2260, D-8500 Nürnberg (DE)**

(72) Erfinder: **Schickaneder, Helmut, Dr. Dipl.Chem., Moosäcker 25, D-8501 Eckental (DE)**
Erfinder: **Engler geb. Hintermayer, Heidrun, Dr. Tierärztin, Ringstrasse 23, D-8501 Cadolzburg (DE)**
Erfinder: **Szelenyi, Istvan, Dr. Arzt, Brahmsstrasse 16, D-8501 Schwaig (DE)**
Erfinder: **Ahrens, Kurt Henning, Dr., Praterstrasse 9, D-8500 Nürnberg (DE)**

(74) Vertreter: **Kraus, Walter, Dr. et al, Patentanwälte Kraus, Weisert & Partner Thomas-Wimmer-Ring 15, D-8000 München 22 (DE)**

**EP 0 124 630 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Pyrimidin-Thioalkylpiridin-Derivate, Verfahren zu ihrer Herstellung und diese Verbindungen enthaltende Arzneimittel.

Die erfindungsgemässen neuen Verbindungen zeichnen sich überraschenderweise durch eine hohe bronchosekretolytische und mukolytische sowie durch eine entzündungshemmende Wirkung aus, die therapeutisch nutzbar ist.

Bekanntlich ist die Viskositätsverringerung des Sputums durch Arzneimittel bei der Behandlung von akuten und chronischen Bronchialerkrankungen (zum Beispiel Infektionen der Atemwege, asthmatischen Beschwerden, obstruktiven Erkrankungen und dgl.) ein wichtiges therapeutisches Ziel. Für diese Krankheitsbilder sind Arzneimittel unterschiedlicher Art bekannt. Sie unterschieden sich durch ihre lokale bzw. systemische Wirksamkeit. Zur lokal wirksamen Arzneimittelgruppe zählt u.a. N-Acetylcystein. Zu den systemisch wirksamen Vertretern — und in der Therapie der Bronchialerkrankung etabliertes Bronchosekretolytikum — gehört zum Beispiel Ambroxol, d.h. Trans-4-(2-Amino-3,5-dibrombenzyl)-aminocyclohexanol. Diese Verbindung wird beispielsweise in der DE-PS 1 593 579 beschrieben.

Pyrimidylsulfide mit antisekretorischer Aktivität und solche Verbindungen enthaltende Arzneimittel werden in der GB-A-2 014 560 beschrieben. Die SU-A-594 116 (referiert in Chemical Abstracts, Bd. 88, 1978, Seite 774, Nr. 190887g) beschreibt bestimmte Pyrimidinthione.

Aufgabe der Erfindung ist es, neue bronchosekretolytische und mukolytisch wirkende Verbindungen zur Verfügung zu stellen, deren orale und parenterale Wirksamkeit gegenüber anerkannt guten Wirkstoffen gleicher Wirkungsrichtung, wie zum Beispiel Ambroxol, wesentlich verbessert ist.

Diese Aufgabe wird durch die Erfindung gelöst.

Gegenstand der Erfindung sind daher neue Pyrimidin-Thioalkylpyridin-Derivate der allgemeinen Formel I

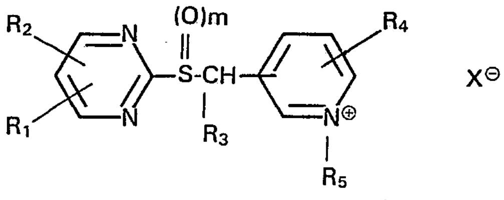 (I)

in der $R_1$ bis $R_4$ unabhängig voneinander für Wasserstoff, Alkylgruppen mit 1 bis 4 Kohlenstoffatomen, Halogen-, Amino- oder Hydroxygruppen stehen, $R_5$ ein freies Elektronenpaar oder eine Alkylgruppe mit 1 bis 4 Kohlenstoffatomen bedeutet, X im Falle, dass $R_5$ eine Alkylgruppe mit 1 bis 4 Kohlenstoffatomen bedeutet, ein Halogenatom darstellt, m den Wert 0 oder 1 hat, wobei die Bindung der Pyrimidin-Thioalkylgruppe in 2-, 3- oder 4-Stellung des Pyridinrings erfolgt ist, sowie ihre therapeutisch verträglichen Säureadditionssalze.

In der allgemeinen Formel I bedeuten $R_1$ und $R_2$ unabhängig voneinander insbesondere Wasserstoffatome, geradkettige oder verzweigtkettige Alkylgruppen mit 1 bis 4 Kohlenstoffatomen, Amino- oder Hxdroxygruppen. Beispiele für Alkylgruppen mit 1 bis 4 Kohlenstoffatomen sind Methylgruppen, Ethylgruppen, Isopropylgruppen und Butylgruppen, vorzugsweise Methylgruppen. Wenn einer der Substituenten $R_1$ und $R_2$ ein Wasserstoffatom ist, dann ist die andere Gruppe in 4- oder 5-Stellung bzw. 5- oder 6-Stellung am Pyrimidinring gebunden, wobei in diesem Fall für $R_1$ die 4-Stellung und $R_2$ die 6-Stellung bevorzugt wird. Wenn beide Gruppen $R_1$ und $R_2$ andere Reste als Wasserstoffatome sind, dann können sie in 4-5, 5-6- oder 4-6-Stellung vorliegen, wobei die 4-6-Stellung bevorzugt wird.

$R_3$ bedeutet in der allgemeinen Formel I insbesondere ein Wasserstoffatom oder eine Alkylgruppe mit 1 bis 4 Kohlenstoffatomen (wie oben definiert), vorzugsweise eine Methylgruppe.

$R_4$ steht insbesondere für ein Wasserstoffatom, ein Halogenatom oder eine Alkylgruppe mit 1 bis 4 Kohlenstoffatomen (wie oben definiert). Halgenatome sind zum Beispiel Fluoratome, Chloratome, Bromatome und Jodatome, vorzugsweise Chloratome.

$R_4$ kann je nach Substitution der Pyrimidin-Thioalkylgruppe (Formel Ia) in den Positionen 2, 3, 4, 5 und 6 des Pyridinrings gebunden sein (d.h. in o-, m-, oder p-Stellung zum Stickstoffatom), wobei die 2- und 6-Stellung (d.h. die o-Stellung) bevorzugt wird.

In der allgemeinen Formel I bedeutet $R_5$ ein freies Elektronenpaar oder eine Alkylgruppe mit 1 bis 4 Kohlenstoffatomen (wie oben definiert), vorzugsweise eine Methyl- oder Ethylgruppe. Im Falle, dass $R_5$ eine Alkylgruppe mit 1 bis 4 Kohlenstoffatomen bedeutet, steht X für ein Halogenatom, wie zum Beispiel Chlor-, Brom- oder Jodatom, vorzugsweise ein Jodatom.

In der allgemeinen Formel I hat m den Wert 0 oder 1. Wie oben definiert, kann die Pyrimidin-Thioalkylgruppe der allgemeinen Formel Ia

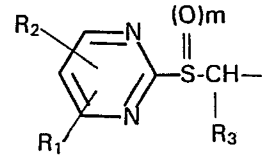 (Ia)

in 2-, 3- oder 4-Stellung des Pyridinrings gebunden sein, wobei die 3-Stellung bevorzugt wird.

Die Verbindungen der allgemeinen Formel I ($R_3$ = H, m = 0, $R_5$ = freies Elektronenpaar) können in verschiedenen tautomeren Formen vorliegen, wie zum Beispiel:

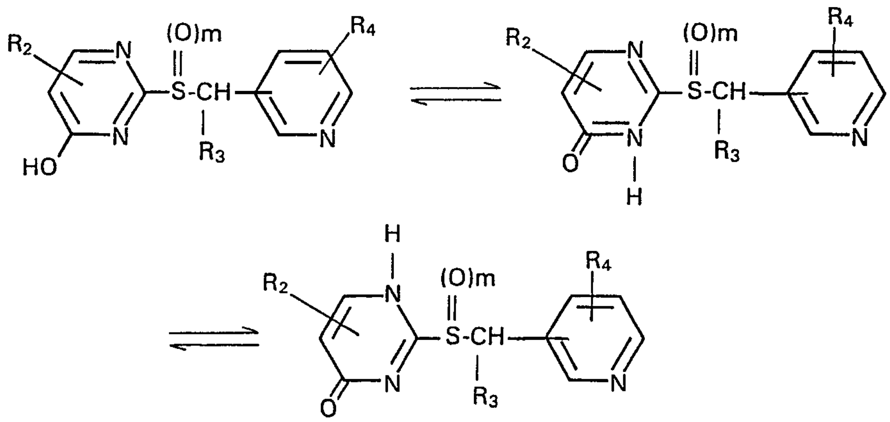

Weiterhin existieren für die Fälle, dass $R_3$ für eine Alkylgruppe mit 1 bis 4 Kohlenstoffatomen steht, m den Wert 0 hat und $R_3$ für Wasserstoff steht und m den Wert 1 hat, Racemate, die durch geeignete chirale Reagenzien, wie zum Beispiel optisch aktive Weinsäure, in ihre Enantiomeren zerlegt werden können.

Im Falle, dass $R_3$ für Alkylgruppen mit 1 bis 4 Kohlenstoffatomen steht und m den Wert 1 hat, existieren Diastereomerenpaare, die, falls gewünscht, durch fraktionierte Kristallisation in die Diastereomeren zerlegt werden können. Deshalb umfassen die erfindungsgemässen Verbindungen auch alle diese oben genannten isomeren Formen.

Eine bevorzugte Gruppe von erfindungsgemässen Pyrimidin-Thioalkylpyridin-Derivaten ist dadurch gekennzeichnet, dass m den Wert 0 hat, $R_1$ und $R_2$ unabhängig voneinander ein Wasserstoffatom, eine Alkylgruppe mit 1 bis 4 Kohlenstoffatomen, eine Halogen-, Amino- oder Hydroxygruppe, die in 3-, 4- oder 5-Stellung am Pyrimidinring gebunden sein können, bedeuten, $R_3$ und $R_4$ jeweils für ein Wasserstoffatom stehen und $R_5$ ein freies Elektronenpaar bedeutet.

Eine weitere bevorzugte Gruppe von erfindungsgemässen Pyrimidin-Thioalkylpyridin-Derivaten ist dadurch gekennzeichnet, dass m den Wert 1 hat, $R_1$ und $R_2$ ein Wasserstoffatom bedeuten und dass $R_5$ ein freies Elektronenpaar ist.

Die Verbindungen der allgemeinen Formel I bilden leicht Säureadditionssalze, zum Beispiel Mono-, Di- und Triadditionssalze, wie beispielsweise Hydrochloride, Hydrobromide, Sulfate, Acetate, Maleinate, Maleate, Fumarate, Oxalate, Methansulfonate, Tartrate, Citrate, Succinate und Embonate usw.

Die erfindungsgemässen Verbindungen (m = 0 und $R_5$ = freies Elektronenpaar) können durch ein Verfahren hergestellt werden, dass dadurch gekennzeichnet ist, dass man in an sich bekannter Weise ein Thiolat der allgemeinen Formel II

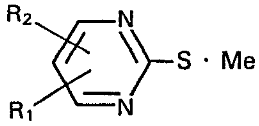

(II)

in der $R_1$ und $R_2$ die oben angegebene Bedeutung hat und Me für ein Alkaliatom steht, mit einem Picolylchlorid-Hydrohalogenid der allgemeinen Formel III

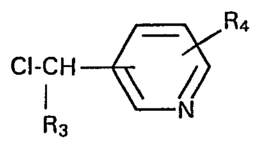

· HHal (III)

worin Hal ein Halogenatom bedeutet und in der die Chlormethylgruppierung in 2-, 3- oder 4-Stellung des Pyridinrests steht und $R_3$ und $R_4$ die oben angegebenen Bedeutungen haben, in der wässrig-alkoholischen Alkalihydroxidlösung, wobei das Alkalihydroxid im Überschuss über die stöchiometrische Menge vorliegt, zu einer Verbindung der allgemeinen Formel IV umsetzt

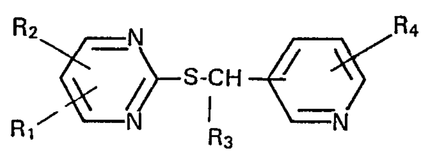

(IV)

und gegebenenfalls die so erhaltene freie Base in ein therapeutisch verträgliches Säureadditionssalz umwandelt.

In der Formel II bedeutet Me ein Alkaliatom, beispielsweise ein Kalium- oder Natriumatom, wobei ein Natriumatom bevorzugt wird.

In der allgemeinen Formel III bedeutet Hal ein Halogenatom, beispielsweise ein Chlor- oder Bromatom, vorzugsweise ein Chloratom. Die Umsetzung findet in einer wässrig-alkoholischen Alkalihydroxidlösung statt, wobei das Alkalihydroxid im Überschuss über die stöchiometrische Menge, vorzugsweise in mindestens der doppelten äquimolaren Menge, vorliegt. Als wässrig-alkoholische Lösung kommt vorzugsweise eine wässrig-ethanolische Lösung in Betracht. Als Alkalihydroxid wird Natriumhydroxid bevorzugt. Bei der Umsetzung geht man zweckmässig so vor, dass man das Thiolat der allgemeinen For-

mel II bei 0 bis 5°C in der wässrig-Alkoholischen Alkalihydroxidlösung auflöst und anschliessend mit der äquimolaren Menge der Picolylchlorid-Hydrohalogenidverbindung, gelöst in wässrig-alkoholischer, vorzugsweise wässrig-ethanolischer, Lösung 2 bis 6 Stunden, vorzugsweise 4 Stunden, bei Raumtemperatur zur Umsetzung bringt.

Aufgrund des angewendeten stöchiometrischen Überschusses des Alkalihydroxids wird die freie Base erhalten, die sodann in üblicher Weise durch Umsetzung mit einer pharmazeutisch annehmbaren Säure in ein therapeutisch verträgliches Salz umgewandelt werden kann.

Die weiterhin erfindungsgemässen Verbindungen (m = 1 und $R_5$ = freies Elektronenpaar) können durch ein Verfahren hergestellt werden, das dadurch gekennzeichnet ist, dass man in an sich bekannter Weise eine Verbindung der allgemeinen Formel IV

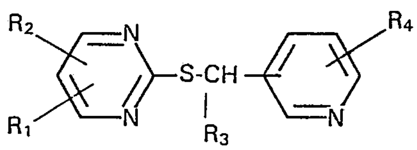

(IV)

in der $R_1$, $R_2$, $R_3$ und $R_4$ die oben angegebenen Bedeutungen haben, in inerten Lösungsmitteln, wie Halogenkohlenwasserstoffen, vorzugsweise Methylenchlorid, mit einem äquimolaren Anteil eines Oxidationsmittels, wie zum Beispiel Kaliumpermanganat, Wasserstoffperoxid oder m-Chlorperbenzoesäure, vorzugsweise m-Chlorperbenzoesäure, umsetzt. Die Umsetzung erfolgt bei 20 bis 30°C, vorzugsweise bei 25°C, indem man innerhalb von 10 bis 15 Minuten einen äquimolaren Anteil von m-Chlorperbenzoesäure, gelöst in Methylenchlorid, zutropft. Die Aufarbeitung der Reaktionslösung und die Isolierung der Verbindungen der allgemeinen Formel V

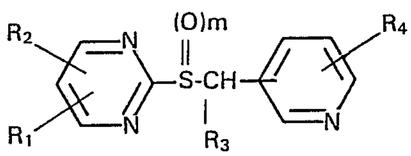

(V)

erfolgen in üblicher Weise. Die erhaltene freie Base wird sodann, wie oben beschrieben, mit einer pharmazeutisch annehmbaren Säure in ein therapeutisch veträgliches Salz umgewandelt.

Die weiterhin erfindungsgemässen Verbindungen (m = 0, $R_5$ = niederes Alkyl, X = Halogen) können durch ein Verfahren hergestellt werden, das dadurch gekennzeichnet ist, dass man in an sich bekannter Weise eine Verbindung der allgemeinen Formel IV

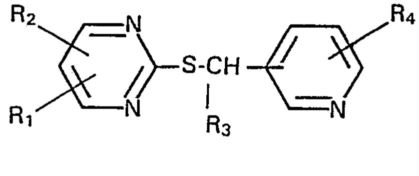

(IV)

in der $R_1$, $R_2$, $R_3$ und $R_4$ die oben angegebenen Bedeutungen haben, in Aceton mit einem äquimolaren Anteil Alkylhalogenid versetzt, vorzugsweise Alkyljodid, und eine Stunde unter Rückflusstemperatur reagieren lässt. Die Isolierung der Verbindung der allgemeinen Formel VI

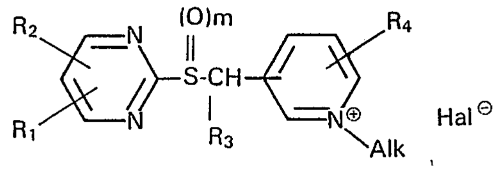

(VI)

in der $R_1$ bis $R_5$, m und X die oben angegebenen Bedeutungen haben, erfolgt durch Einengen der Reaktionslösung und Kristallisation des entstandenen Produkts in einem alkoholischen Lösungsmittel, vorzugsweise in Isopropanol.

Die erfindungsgemässen Verbindungen zeichnen sich durch eine überraschend verbesserte bronchosekretolytische und mukolytische Wirkung aus, so dass sie für die Therapie in wesentlich kleineren Mengen als bekannte Wirkstoffe dosiert werden können. Zusätzlich konnte bei den erfindungsgemässen Verbindungen ein antiphlogistisches Wirkprofil nachgewiesen werden.

So zeigen zum Beispiel Verbindungen der allgemeinen Formel I, bei denen $R_1$ eine in 4-Stellung des Pyrimidinrings gebundene Hydroxygruppe ist, $R_2$ ein Wasserstoffatom darstellt oder $R_1$ und $R_2$ jeweils eine in 4- bzw. 6-Stellung des Pyrimidinrings gebundene Aminogruppe darstellen, $R_3$ und $R_4$ jeweils ein Wasserstoffatom sind, $R_5$ ein freies Elektronenpaar darstellt und m den Wert 0 hat, wobei die Pyrimidin-Thioalkylgruppe in 3-Stellung des Pyrimidinrings gebunden ist, eine antiphlogistische Aktivität. Demgemäss könnte die Verwendung dieser Verbindungen als antiphlogistische, d.h. entzündungshemmende, Mittel in Betracht gezogen werden.

Ein weiterer Gegenstand der Erfindung ist daher eine pharmazeutische Zubereitung bzw. ein Arzneimittel mit bronchosekretolytischer und mukolytischer Wirkung, die bzw. das neben üblichen Hilfs- und Trägerstoffen wenigstens ein Pyrimidin-Thioalkylpyridin-Derivat der allgemeinen Formel I enthält.

Die erfindungsgemässe pharmazeutische Zubereitung kann für alle Arten von Bronchialerkrankungen, beispielsweise akute und chronische Atemwegserkrankungen, zur Nachbehandlung von chirurgischen Eingriffen in den Atemwegen sowie bei allen Prozessen, bei denen eine Verflüssigung des Bronchialschleims wünschenswert ist, verwendet werden.

Die erfindungsgemäss verwendete Verbindung wird vorzugsweise oral verabreicht. Gewöhnlich beträgt die orale Tagesdosis 0,01 bis 0,2 g, vorzugsweise 0,02 bis 0,1 g, die in einer oder mehreren Tagesdosen verabreicht werden kann.

Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten

Mindestmenge ausgekommen werden kann, während in anderen Fällen die genannte obere Grenze überschritten werden muss. Im Falle der Applikation grösserer Mengen kann es empfehlenswert sein, diese in mehreren Einzelgaben über den Tag zu verteilen.

Für die orale Verabreichung kann der Wirkstoff beispielsweise in Form von Kapseln formuliert werden, die durch herkömmliche Massnahmen mit pharmazeutisch annehmbaren Exzipientien, z.B. Bindemitteln (wie vorgelatinisierter Maisstärke, Polyvinylpyrrolidon oder Hydroxypropylmethylcellulose); Füllstoffen (wie Lactose, mikrokristalline Cellulose oder Calciumphosphat); Schmiermitteln (wie Magnesiumstearat, Talk oder Kieselsäure); Sprengmitteln (z.B. Kartoffelstärke oder Natriumstärkeglykollat); oder Befeuchtungsmitteln (z.B. Natriumlaurylsulfat), hergestellt werden. Die Kapseln können nach bekannten Methoden beschichtet werden. Flüssige Zubereitungen für die orale Verabreichung oder für ein direktes Einträufeln können beispielsweise die Form von Lösungen, Sirups oder Suspensionen haben, oder sie können als Trockenprodukt zur Rekonstitution mit Wasser oder einem anderen geeigneten Träger vor dem Gebrauch präsentiert werden. Solche flüssigen Zubereitungen können durch herkömmliche Masnahmen mit pharmazeutisch annehmbaren Additiven, z.B. Suspendierungsmitteln (wie Sorbitsirup, Methylcellulose oder hydrierten essbaren Fetten); Emulgierungsmitteln (z.B. Lecithin oder Acacia); nichtwässrigen Trägern (z.B. Mandelöl, ölige Ester oder Ethylalkohol); und Konservierungsmitteln (z.B. Methyl- oder Propyl-p-hydroxybenzoaten oder Sorbinsäure) hergestellt werden.

Für die bukkale Verabreichung können die Zubereitungen in Form von Tabletten oder Lutschpastillen, die auf herkömmliche Weise formuliert werden, vorliegen.

Die erfindungsgemäss verwendete Verbindung kann für die parenterale Verabreichung durch Injektion oder für die Infusion formuliert werden. Zubereitungen für die Injektion können in Einheitsdosisform, z.B. in Ampullen oder in Viel-Dosen-Behältern, mit einem zugegebenen Konservierungsstoff vorliegen. Die Zubereitungen können auch solche Formen, wie Suspensionen, Lösungen oder Emulsionen in öligen oder wässrigen Trägern einnehmen, und sie können Formulierungsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, z.B. sterilem, pyrogenfreiem Wasser, vor dem Gebrauch vorliegen.

Zur Verabreichung durch Inhalieren wird die erfindungsgemäss verwendete Verbindung geeigneterweise in Form eines Aerosolsprays aus unter Druck gesetzten Packungen oder Zerstäubern unter Verwendung eines geeigneten Treibmittels, z.B. Dichlordifluormethan, Trichlorfluormethan, Dichlortetrafluorethan, Kohlendioxid oder eines anderen geeigneten Gases, abgegeben. Im Falle eines unter Druck gesetzten Aerosols kann die Dosiseinheit in der Weise bestimmt werden, dass ein Ventil zur Abgabe einer dosierten Menge vorgesehen ist.

Pharmakologische Untersuchungen haben ergeben, dass die erfindungsgemäss verwendeten Pyrimidin-Thioalkylpyridin-Derivate gegenüber dem bekannten Vergleichsprodukt Ambroxol überlegene bronchosekretolytische und mukolytische Eigenschaften aufweisen.

Im einzelnen wurden folgende Untersuchungen durchgeführt.

1. *Pharmakodynamik*

1.1. *Sekretstimulierende Aktivität*

Bronchosekretolytisch wirksame Verbindungen fördern die tracheale Ausscheidung von Phenolrot (Chronic Bronchitits Research Group, Chinese Medical Journal 3 : 259, 1977).

Die Steigerung der trachealen Phenolrot-Ausscheidung ist ein Mass der bronchosekretolytischen Wirkung. Diese Wirkung der Prüfsubstanzen wurde nach oraler Verabreichung an wachen Mäusen untersucht. Die in der Tabelle I angegebenen $ED_{50}$-Werte wurden anhand der Regressionskurven berechnet.

TABELLE I

| Verbindung | $ED_{50}$ (mg/kg) |
|---|---|
| Ambroxol | 250 |
| Beispiel 1 | 5 |
| Beispiel 2 | 100 |
| Beispiel 3 | 2 |
| Beispiel 4 | >100 |
| Beispiel 5 | 15 |
| Beispiel 6 | 2 |
| Beispiel 7 | 15 |
| Beispiel 8 | >100 |
| Beispiel 9 | 2 |
| Beispiel 10 | 100 |
| Beispiel 11 | 10 |
| Beispiel 12 | 1 |
| Beispiel 13 | 70 |

1.2. *Entzündungshemmende Aktivität*

Die entzündungshemmenden Eigenschaften der erfindungsgemässen Verbindungen wurden mit Hilfe des Carrageenin-Ödem-Tests an männlichen Ratten (130-160 g Durchschnittsgewicht, SIV 50, Fa. Ivanovas, Kisslegg) durchgeführt. Dabei wurde die Hemmung der durch Carrageenin gesetzten Entzündung (Ödembildung) an der rechten Hinterpfote gemessen. Die Prüfsubstanzen wurden 30 Minuten vor der Carrageenininjektion mit einer Schlundsonde intragastral verabreicht. Die Ergebnisse sind in Tabelle II angegeben. [Higgs et al. European Journal of Pharmacology 66, 81-86 (1980)].

TABELLE II

| Verbindung | Appl. Menge mg/kg i.g. | Hemmung in % |
|---|---|---|
| Beispiel 2 | 100 | 45 |
| Beispiel 4 | 100 | 30 |

Die Erfindung wird in den Beispielen erläutert.

*Beispiel 1*

*Herstellung von 4-Amino-2-(pyridyl-3-methylthio)--pyrimidin*

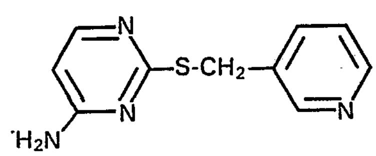

Zu einer Lösung aus 12,7 g (0,1 mol) 4-Amino-2--mercaptopyrimidin in 250 ml Ethanol werden bei 0°C 8,4 g (0,21 mol) Natriumkydroxid, gelöst in 120 ml Wasser, zugetropft. Anschliessend werden 16,4 g (0,1 mol) 3-Picolylchlorid-Hydrochlorid, gelöst in 100 ml Wasser, langsam zugegeben und 4 Stunden bei Raumtemperatur gerührt. Die Reaktionslösung wird eingeengt, in 500 ml Ether aufgenommen, die organische Phase dreimal mit je 100 ml Wasser gewaschen, über Natriumsulfat getrocknet, abfiltriert und das Filtrat im Vakuum eingeengt. Der anfallende Feststoff wird aus Essigsäureethylester umkristallisiert.

Farblose Kristalle vom Schmelzpunkt 119-120°C
Rf = 0,40 ($CH_2Cl_2$/MeOH 9:1)

Ausbeute: 12,4 g (57%)
$C_{10}H_{10}N_4S$ (218)

| | | | |
|---|---|---|---|
| Ber.: | C 55,03 | H 4,62 | N 25,67 |
| Gef.: | C 55,14 | H 4,63 | N 25,80 |

$^1$H-NMR-Daten: ($d_6$-DMSO, TMS als interner Standard)
δ = 4,33 (s) (S-C$\underline{H}_2$) 2 H, 6,17 (d) (Aromaten-$\underline{H}$) 1 H, 7,00 (s) (-N$\underline{H}_2$, austauschbar mit $D_2O$) 2 H, 7,33 (dd) (Aromaten-$\underline{H}$) 1 H, 7,83 (m) (Aromaten-$\underline{H}$) 1 H, 7,93 (d) (Aromaten-$\underline{H}$) 1 H, 8,43 (m) (Aromaten-$\underline{H}$) 1 H, 8,67 (s) (Aromaten-$\underline{H}$) 1 H ppm.

*Beispiel 2*

*Herstellung von 4-Hydroxy-2-(pyridyl-3-methylthio)-pyrimidin*

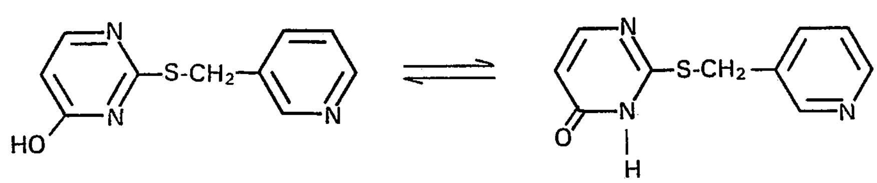

Die Herstellung erfolgt analog Beispiel 1 aus 4-Hydroxy-2-mercapto-pyrimidin und 3-Picolylchlorid-Hydrochlorid.

Farblose Kristalle vom Schmelzpunkt 167-168°C

Rf = 0,31 ($CH_2Cl_2$/MeOH 9 : 1)

Ausbeute: 6,8 g (32%)
$C_{10}H_9N_3OS$ (213)

| | | | |
|---|---|---|---|
| Ber.: | C 54,78 | H 4,14 | N 19,16 |
| Gef.: | C 54,71 | H 4,19 | N 19,22 |

$^1$H-NMR-Daten: ($d_6$-DMSO, TMS als interner Standard)
δ = 4,47 (s) (-S-C$\underline{H}_2$) 2 H, 6,17 (d) (Aromaten-$\underline{H}$) 1 H, 7,37 (dd) (Aromaten-$\underline{H}$) 1 H, 7,77-8,10 (m) (Aromaten-$\underline{H}$) 2 H, 8,50 (d) (Aromaten-$\underline{H}$) 1 H, 8,70 (s) (Aromaten-$\underline{H}$) 1 H, 12,66 (s, breit) (-O$\underline{H}$) (austauschbar mit $D_2O$) 1 H ppm.

*Beispiel 3*

*Herstellung von 4-Amino-6-hydroxy-2-(pyridyl-3-methylthio)-pyrimidin*

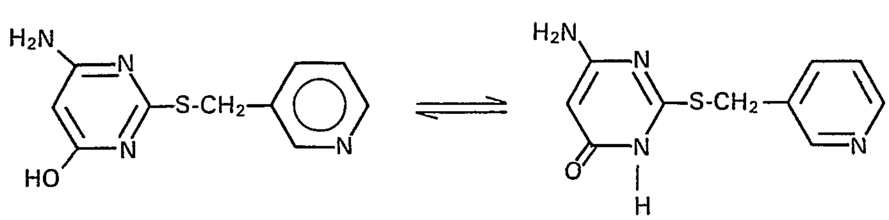

Die Herstellung erfolgt analog Beispiel 1 aus 4-Amino-6-hydroxy-2-mercaptopyrimidin und 3-Picolylchlorid-Hydrochlorid.

Farblose Kristalle vom Schmelzpunkt 210-211 °C.

Rf = 0,57 ($CH_2Cl_2$/MeOH 8 : 2)

Ausbeute: 13,3 g (57%)

$C_{10}H_{10}N_4OS$ (234)

| | | | |
|---|---|---|---|
| Ber.: | C 51,27 | H 4,30 | N 23,91 |
| Gef.: | C 51,37 | H 4,35 | N 24,03 |

$^1$H-NMR-Daten: ($d_6$-DMSO, TMS als interner Standard)
δ = 4,37 (s) (S-$CH_2$) 2 H, 5,03 (s) (>=CH) 1 H, 6,60 (s) (-$NH_2$) (austauschbar mit $D_2O$) 2 H, 7,33 (dd) (Aromaten-H) 1 H, 7,87 (d) (Aromaten-H) 1 H, 8,70 (s) (Aromaten-H) 1 H, 11,50 (s) (N-H) (austauschbar mit $D_2O$) 1 H ppm.

*Beispiel 4*

*Herstellung von 4,6-Diamino-2-(pyridyl-3-methylthio)-pyrimidin-Succinat*

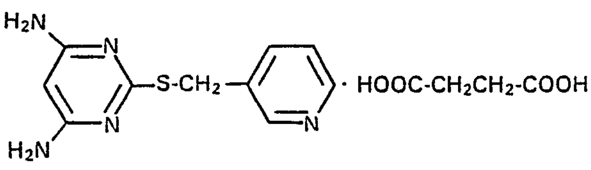

Die Herstellung erfolgt analog Beispiel 1 aus 4,6-Diamino-2-mercaptopyrimidin und 3-Picolylchlorid-Hydrochlorid. Die Herstellung des Succinats erfolgt durch Versetzen einer ethanolischen Lösung aus 4,6-Diamino-2-(pyridyl-3-methylthio)-pyrimidin mit einer äquimolaren 10%igen ethanolischen Bernsteinsäurelösung. Dabei fällt das Succinat analysenrein an.

Farblose Kristalle vom Schmelzpunkt 149-150°C

Rf = 0,22 ($CH_2Cl_2$/MeOH 95 : 5 $NH_3$-Dämpfe)

Ausbeute: 15,1 g (43%)

$C_{14}H_{17}N_5O_5S$ (351)

| | | | |
|---|---|---|---|
| Ber.: | C 47,86 | h 4,88 | N 19,93 |
| Gef.: | C 47,80 | H 4,92 | N 19,81 |

$^1$H-NMR-Daten: ($d_6$-DMSO, TMS als interner Standard)

δ = 2,43 (s) [-$(CH_2)_2$-] 4 H, 4,23 (s) (-S-$CH_2$) 2 H, 5,20 (s) (Aromaten-H) 1 H, 6,13 (s) (2-$NH_2$) (austauschbar mit $D_2O$) 4 H, 7,27 (dd) (Aromaten-H) 1 H, 7,83 (d) Aromaten-H) 1 H, 8,43 (d) (Aromaten-H) 1 H, 8,67 (s) (Aromaten-H) 1 H, 12,20 (s, breit) (-COOH) (austauschbar mit $D_2O$) 2 H ppm.

*Beispiel 5*

*Herstellung von 4-Hydroxy-6-methyl-2-(pyridyl-3-methylthio) pyrimidin*

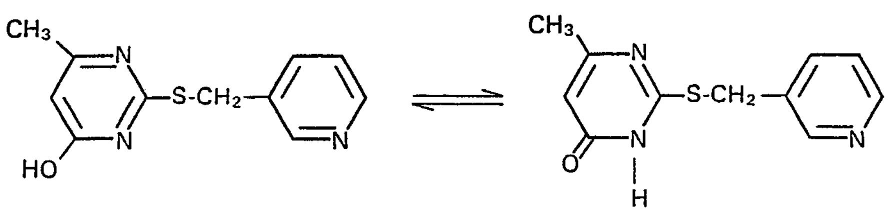

Die Herstellung erfolgt analog Beispiel 1 aus 4-Hydroxy-6-methyl-2-mercaptopyrimidin und 3-Picolylchlorid-Hydrochlorid.

Farblose Kristalle vom Schmelzpunkt 168-169°C

Rf = 0,41 ($CH_2Cl_2$/MeOH 9 : 1)

Ausbeute: 15,8 g (68%)

$C_{11}H_{11}N_3OS$ (233)

| | | | |
|---|---|---|---|
| Ber.: | C 56,63 | H 4,75 | N 18,01 |
| Gef.: | C 56,69 | H 4,89 | N 17,99 |

$^1$H-NMR-Daten: ($d_6$-DMSO, TMS als interner Standard)
δ = 2,23 (s) (-$CH_3$) 3 H, 4,43 (s) (S-$CH_2$) 2 H, 6,07 (s) (Aromaten-H) 1 H, 7,40 (dd) (Aromaten-H) 1 H, 7,87 (m) (Aromaten-H) 1 H, 8,47 (d) (Aromaten-H) 1 H, 8,70 (s) (Aromaten-H) 1 H ppm.

*Beispiel 6*

*Herstellung von 4-Amino-6-hydroxy-2-(pyridyl-6-chlor-3-methylthio) pyrimidin*

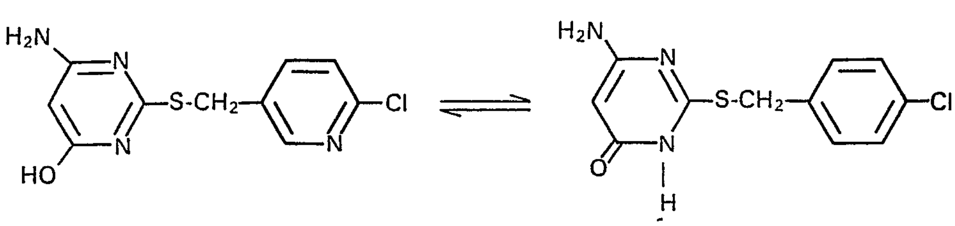

Die Herstellung erfolgt analog Beispiel 1 aus 4-Amino-6-hydroxy-2-mercaptopyrimidin und 3-Picolylchlorid-Hydrochlorid.

Hellgelbe Kristalle vom Schmelzpunkt 227-228°C

Rf = 0,35 ($CH_2Cl_2$/M2OH 9 : 1)

Ausbeute: 11,0 g (41%)

$C_{10}H_9$ $ClN_4OS$ (269)

$^1$H-NMR-Daten: ($d_6$-DMSO, TMS als interner Standard)
δ = 4,27 (s) (S-$CH_2$) 2 H, 5,00 (s) (> =CH) 1 H, 6,57 (s) (-$NH_2$) (austauschbar mit $D_2O$) 2 H, 7,43 (d) (Aromaten-H) 1 H, 7,97 (dd) (Aromaten-H) 1 H, 8,53 (s) (Aromaten-H) 1 H ppm.

*Beispiel 7*

*Herstellung von 2-[Pyridyl-3-(2-ethylthio)] pyrimidin-Oxalat*

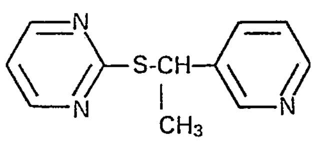

· 3/2 $(HOOC)_2$

Die Herstellung erfolgt analog Beispiel 1 aus 2-Mercaptopyrimidin und 3-(2-Chlorethyl)pyrimidin-Hydrochlorid. Die Herstellung des Oxalats erfolgt analog Beispiel 4.

Farblose Kristalle vom Schmelzpunkt 115-116°C

Rf = 0,71 ($CH_2Cl_2$/MeOH 95 : 5 $NH_3$-Dämpfe)

Ausbeute: 10,9 g (31%)

$C_{14}H_{14}N_3O_6S$ (352)

| | | | |
|---|---|---|---|
| Ber.: | C 47,73 | H 4,01 | N 11,93 |
| Gef.: | C 47,81 | H 3,99 | N 11,92 |

$^1$H-NMR-Daten: ($d_6$-DMSO, TMS als interner Standard)
δ = 1,77 (d) ($CH_3$) 3 H, 5,10 (q) (S-CH) 1 H, 7,23 (t) (Aromaten-H) 1 H, 7,50 (dd) (Aromaten-H) 1 H, 8,07 (m) (Aromaten-H) 1 H, 8,43-8,93 (m) (Aromaten-H) 4 H ppm.

*Beispiel 8*

*Herstellung von 2-[Pyridyl-3-(2-ethylthio)-6-methyl]-pyrimidin*

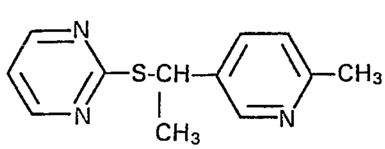

Die Herstellung erfolgt analog Beispiel 1 aus 2-Mercaptopyrimidin und 3-(2-Chlorethyl-6-methyl)pyrimidin-Hydrochlorid.

Hellgelbes Öl

Rf = 0,39 ($CH_2Cl_2$/MeOH 95 : 5)

Ausbeute: 13,4 g (58%)

$C_{12}H_{13}N_3S$ (231)

$^1$H-NMR-Daten: ($CDCl_3$, TMS als interner Standard)
δ = 1,73 (d) (CH-$CH_3$) 3 H, 2,50 (s) (-CH) 3 H, 5,03 (q) (-CH-$CH_3$) 1 H, 6,90 (t) (Aromaten-H) 1 H, 7,10 (d) (Aromaten-H) 1 H, 7,70 (dd) (Aromaten-H) 1 H, 8,47 (d) (Aromaten-H) 2 H, 8,67 (s) (Aromaten-H) 1 H ppm.

*Beispiel 9*

*Herstellung von 2-(Pyridyl-3-methylsulfinyl)-pyrimidin-Hydrochlorid*

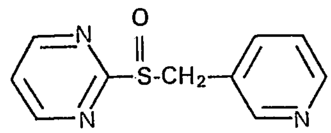

· HCl

Zu einer Lösung aus 20,3 g (0,1 mol) 2-(Pyridyl)--3-methylthio)-pyrimidin in 500 ml Methylenchlorid werden bei Raumtemperatur innerhalb von 10-15 Minuten 18,1 g (0,11 mol) m-Chlorperbenzoesäure, gelöst in 300 ml Methylenchlorid, zugetropft. Nach einer Reaktionszeit von 15 Minuten versetzt man die Reaktionslösung mit 120 ml 3%iger Natriumhydrogencarbonat-Lösung und trennt die Methylenchloridphase ab. Die organische Phase wird mit Wasser neutralgewaschen, über Natriumsulfat getrocknet und im Vakuum eingeengt. Die anfallende Base wird mit einer 200 ml ethanolischer äquimolarer HCl-Lösung versetzt und das Hydrochlorid mit Ether ausgefällt. Das 2-(Pyridyl-3-methylsulfinyl)-pyrimidin--Hydrochlorid wird aus Isopropanol umkristallisiert.

Farblose Kristalle vom Schmelzpunkt 168-169°C

Rf = 0,27 ($CH_2Cl_2$/MeOH 95 : 5 $NH_3$-Dämpfe)

Ausbeute: 8,2 g (32%)

$C_{10}H_{10}ClN_3OS$ (256)

| | | | |
|---|---|---|---|
| Ber.: | C 46,97 | H 3,94 | N 16,43 |
| Gef.: | C 46,91 | H 3,97 | N 16,25 |

$^1$H-NMR-Daten: ($d_4$-MeOH, TMS als interner Standard)
δ = 4,83 (dd) (SO-$CH_2$) 2 H, 7,53-9,27 (m) (Aromaten-H) 7 H ppm.

*Beispiel 10*

*Herstellung von 2-(Pyridyl-4-methylsulfinyl)-pyrimidin-Hydrochlorid*

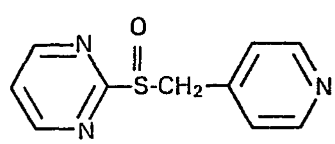

· HCl

Die Herstellung erfolgt analog Beispiel 9 aus 2-(Pyridyl-4-methylthio)-pyrimidin und m-Chlorperbenzoesäure.

Farblose Kristalle vom Schmelzpunkt 165-166°C

Rf = 0,20 ($CH_2Cl_2$/MeOH 95 : 5 $NH_3$-Dämpfe)

Ausbeute: 7,65 g (30%)

$C_{10}H_{10}ClN_3OS$ (256)

$^1$H-NMR-Daten: ($d_6$-DMSO, TMS als interner Standard)
δ = 4,80 (dd) (SO-$CH_2$) 2 H, 7,73 (M) (Aromaten-H) 3 H, 8,83 (d) (Aromaten-H) 2 H, 9,00 (d) (Aromaten-H) 2 H ppm.

*Beispiel 11*

*Herstellung von 4-Methyl-2-(pyridyl-3-methylsulfinyl)-pyrimidin-Oxalat*

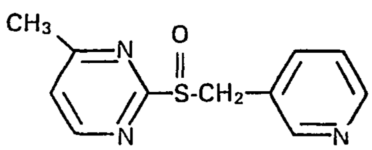 $\times$ $(COOH)_2$

Die Herstellung erfolgt analog Beispiel 9 aus 3-Methyl-2-mercapto-pyrimidin und m-Chlorperbenzoesäure.

Farblose Kristalle vom Schmelzpunkt 132-134°C

Rf = 0,81 ($CH_2Cl_2$/MeOH 8 : 2 $NH_3$-Dämpfe)

Ausbeute: 11,3 g (35%)

$C_{13}H_{13}N_3O_5S$ (323)

$^1$H-NMR-Daten: ($d_6$-DMSO, TMS als interner Standard)
δ = 2,53 (s) (-$CH_3$) 3 H, 4,43 (dd) (SO-$CH_2$) 2 H, 7,17-7,67 (m) (Aromaten-H) 3 H, 8,00-8,67 (m) (Aromaten-H) 2 H, 8,77 (d) (Aromaten-H) 1 H ppm.

*Beispiel 12*

*Herstellung von 2-(3-Methylthio-1-methylpyridinium)-pyrimidin-Jodid*

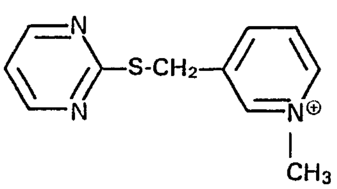 $J^{\ominus}$

Zu einer Lösung aus 20,3 g (0,1 mol) 2-(Pyridyl-3-methylthio)-pyrimidin in 250 ml Aceton gibt man 14,2 g (0,1 mol) Methyljodid und lässt 1 Stunde bei Rückflusstemperatur reagieren. Anschliessend engt man die Reaktionslösung im Vakuum zur Trockene ein und kristallisiert den anfallenden hellgelben Feststoff aus Isopropanol um.

Hellgelber Feststoff von Schmelzpunkt 104-105°C

Rf = 0,38 ($CH_2Cl_2$/MeOH 8 : 2 $NH_3$-Dämpfe)

Ausbeute: 30,0 g (87%)

$C_{11}H_{12}JN_3S$ (345)

| | | | | | | |
|---|---|---|---|---|---|---|
| Ber.: | C | 38,27 | H | 3,50 | N | 12,17 |
| Gef.: | C | 38,44 | H | 3,49 | N | 12,16 |

$^1$H-NMR-Daten: ($d_4$-MeOH, TMS als interner Standard)
δ = 4,67 (s) (S-$CH_2$) 2 H, 7,20 (t) (Aromaten-H) 1 H, 8,13 (dd) (Aromaten-H) 1 H, 8,57-9,0 (m) (Aromaten-H) 4 H, 9,30 (s) (Aromaten-H) 1 H ppm.

*Beispiel 13*

*Herstellung von 2-(3-Methylthio-1-ethyl-pyridinium)-pyrimidin-Jodid*

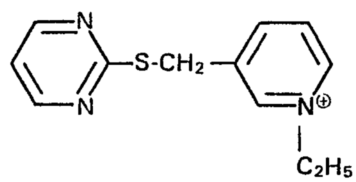 $J^{\ominus}$

Die Herstellung erfolgt analog Beispiel 12 aus 2-(Pyridyl-3-methylthio)-pyrimidin und Ethyljodid.

Hellgelber Feststoff vom Schmelzpunkt 137-138°C

Rf = 0,15 ($CH_2Cl_2$/MeOH 8 : 2)

Ausbeute: 24,0 g (67%)

$C_{12}H_{14}JN_3S$ (359)

| | | | | | | |
|---|---|---|---|---|---|---|
| Ber.: | C | 40,12 | H | 3,93 | N | 11,70 |
| Gef.: | C | 40,12 | H | 3,97 | N | 11,76 |

$^1$H-NMR-Daten: ($d_6$-DMSO, TMS als interner Standard)
δ = 1,60 (t) ($CH_3$-$CH_2$-) 3 H, 4,67 (s) (S-$CH_2$) 2 H, 4,73 (q) ($CH_2$-$CH_3$) 2 H, 7,33 (t) (Aromaten-H) 1 H, 8,20 (dd) (Aromaten-H) 1 H, 8,80 (m) (Aromaten-H) 3 H, 9,10 (d) (Aromaten-H) 1 H, 9,40 (s) (Aromaten-H) 1 H ppm.

*Beispiel 14*

*Herstellung von den erfindungsgemässen Wirkstoff enthaltenden Weichgelatinekapseln*

*Zusammensetzung:*

| | |
|---|---|
| z.B. Erfindungsgemässe Verbindung Beispiel 12 | 100,0 mg |
| Rübol | 281,0 mg |
| Bienenwachs | 2,0 mg |
| Partiell hydriertes Pflanzenöl | 8,0 mg |
| Sojalecithin | 8,0 mg |
| 3-Ethoxy-4-hydroxy-benzaldehyd | 1,0 mg |
| | 400,0 mg |

Die Substanzen werden gemischt, homogenisiert und in üblicher Weise zu Weichgelatinekapseln verarbeitet.

*Beispiel 15*

*Herstellung von den erfindungsgemässen Wirkstoff enthaltenden Dosier-Aerosol*

*Zusammensetzung:*

| | |
|---|---|
| z.B. Erfindungsgemässe Verbindung Beispiel 12 | 10,0 mg |
| Sorbitantrioleat | 0,5 mg |
| Difluormethan | 35,5 mg |
| Dichlortetrafluorethan | 25,0 mg |
| Gesamtdosis pro Hub | 71,0 mg |

Die Substanzen werden kalt gelöst und 10 g Lösung in eine geeignete Druckgasverpackung eingebracht.

*Beispiel 16*

*Herstellung von den erfindungsgemässen Wirkstoff enthaltenden Ampullen*

*Zusammensetzung:*

| | |
|---|---|
| z.B. Erfindungsgemässe Verbindung Beispiel 12 | 100,0 mg |
| Polyethylenglykol 300 | 630,0 mg |
| 1,2-Propandiol | 735,0 mg |
| α-Toxopherol | 1,0 mg |
| Dinatriumhydrogenphosphat | 4,0 mg |
| Natriumdihydrogenphosphat | 20,0 mg |
| Wasser für Injektionszwecke | 610,0 mg |
| Gesamtfüllgewicht | 2 100,0 mg |

Die Substanzen werden gelöst und die Lösung in üblicher Weise zu Ampullen à 2 ml verarbeitet.

**Patentansprüche für die Vertragsstaaten:**
BE, CH, DE, FR, GB, IT, LI, LU, NL, SE

1. Pyrimidin-Thioalkylpyridin-Derivate der allgemeinen Formel I

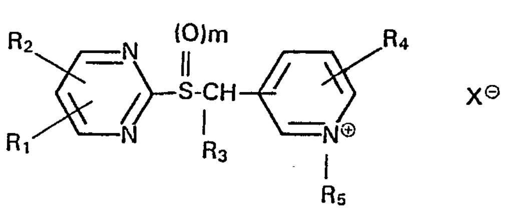

(I)

in der $R_1$ bis $R_4$ unabhängig voneinander für Wasserstoff, $C_1$-$C_4$-Alkyl-, Halogen-, Amino- oder Hydroxygruppen stehen, $R_5$ ein freies Elektronenpaar oder eine $C_1$-$C_4$-Alkylgruppe bedeutet, X im Falle, dass $R_5$ eine $C_1$-$C_4$-Alkylgruppe bedeutet, ein Halogenatom darstellt, m den Wert 0 oder 1 hat, wobei die Bindung der Pyrimidin-Thioalkylgruppe in 2-, 3 oder 4-Stellung des Pyridinrings erfolgt ist, sowie ihre therapeutisch verträglichen Säureadditionssalze.

2. Pyrimidin-Thioalkylpyridin-Derivate nach Anspruch 1, dadurch gekennzeichnet, dass m den Wert 0 hat, $R_1$ und $R_2$ unabhängig voneinander ein Wasserstoffatom, eine $C_1$-$C_4$-Alkyl-, Halogen-, Amino- oder Hydroxygruppe, die in 3-, 4- oder 5-Stellung am Pyrimidinring gebunden sein können, bedeuten, $R_3$ und $R_4$ jeweils für ein Wasserstoffatom stehen und $R_5$ ein freies Elektronenpaar bedeutet.

3. Pyrimidin-Thioalkylpyridin-Derivate nach Anspruch 1, dadurch gekennzeichnet, dass $R_3$ für eine $C_1$-$C_4$-Alkylgruppe steht und dass $R_4$ ein Wasserstoffatom oder eine $C_1$-$C_4$-Alkylgruppe bedeutet.

4. Pyrimidin-Thioalkylpyridin-Derivate nach Anspruch 1 oder 3, dadurch gekennzeichnet, dass m den Wert 1 hat, $R_1$ und $R_2$ ein Wasserstoffatom bedeuten und dass $R_5$ ein freies Elektronenpaar ist.

5. Pyrimidin-Thioalkylpyridin-Derivate nach einem der Ansprüche 1 bis 3, dadurch gekennzeichnet, dass m den Wert 0 hat, $R_5$ für eine $C_1$-$C_4$-Alkylgruppe steht und dass X ein Halogenatom bedeutet.

6. Verfahren zur Herstellung von Pyrimidin-Thioalkylpyridin-Derivaten nach Anspruch 1, dadurch gekennzeichnet, dass man

a) zur Herstellung von Verbindungen der allgemeinen Formel I, bei denen m den Wert 0 hat, eine Verbindung der Formel II

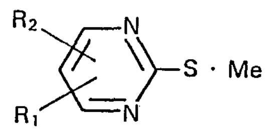

(II)

worin $R_1$ und $R_2$ die in Anspruch 1 angegebene Bedeutung haben und Me für ein Alkaliatom steht, mit einem Picolylchlorid-Hydrohalogenid der allgemeinen Formel III

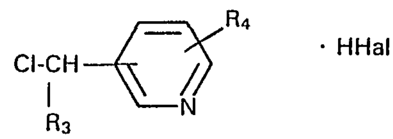

(III)

worin Hal ein Halogenatom bedeutet und in der die Chlormethylgruppierung in 2-, 3- oder 4-Stellung des Pyridinrestes steht und $R_3$ und $R_4$ die in Anspruch 1 angegebene Bedeutung haben,
in der wässrig-alkoholischen Alkalihydroxidlösung, wobei das Alkalihydroxid im Überschuss über die stöchiometrische Menge vorliegt, umsetzt oder dass man

b) zur Herstellung von Verbindungen, bei denen m den Wert 1 hat, eine Verbindung der allgemeinen Formel IV

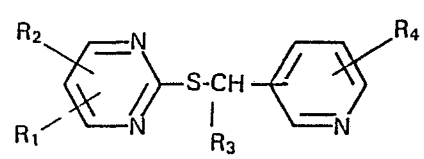

(IV)

in der $R_1$ bis $R_4$ die in Anspruch 1 angegebene Bedeutung haben,
in einem inerten Lösungsmittel mit einem äquimolaren Anteil eines Oxidationsmittels versetzt und die erhaltene Sulfinylverbindung der allgemeinen Formel V

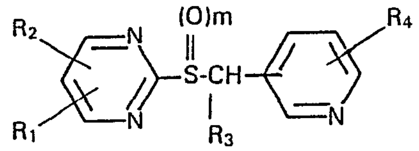

(V)

isoliert oder

c) zur Herstellung von Verbindungen, bei denen m den Wert 0 hat, ein Pyrimidin-Thioalkylpyridin-Derivat der allgemeinen Formel IV

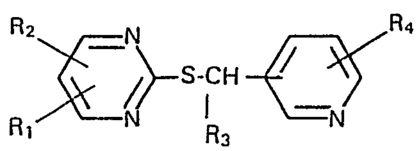

(IV)

in der $R_1$ bis $R_4$ die in Anspruch 1 angegebenen Bedeutungen haben,
in einem Lösungsmittel mit einer äquimolaren Menge eines Alkylhalogenids zu einer Verbindung der allgemeinen Formel VI

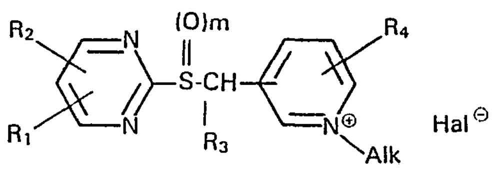

(VI)

umsetzt und

d) gegebenenfalls die in a) bis c) erhaltene Verbindung in ein physiologisch annehmbares Salz umwandelt.

7. Arzneimittel, dadurch gekennzeichnet, dass es neben üblichen Hilfs- und Trägerstoffen wenigstens ein Pyrimidin-Thioalkylpyridin-Derivat nach einem der Ansprüche 1 bis 6 enthält.

**Patentansprüche für den Vertragsstaat:** AT

1. Verfahren zur Herstellung von Pyrimidin-Thioalkylpyridin-Derivaten der allgemeinen Formel I

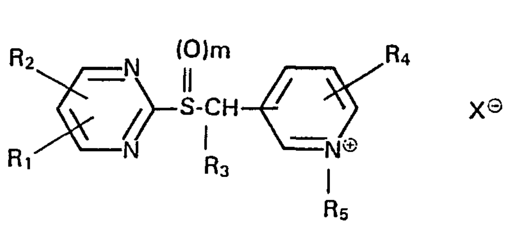

(I)

in der $R_1$ bis $R_4$ unabhängig voneinander für Wasserstoff, $C_1$-$C_4$-Alkyl-, Halogen-, Amino- oder Hydroxygruppen stehen, $R_5$ ein freies Elektronenpaar oder eine $C_1$-$C_4$-Alkylgruppe bedeutet, X im Falle, dass $R_5$ eine $C_1$-$C_4$-Alkylgruppe bedeutet, ein Halogenatom darstellt, m den Wert 0 oder 1 hat, wobei die Bindung der Pyrimidin-Thioalkylgruppe in 2-, 3- oder 4-Stellung des Pyridinrings erfolgt ist, sowie ihrer therapeutisch verträglichen Säureadditionssalze, dadurch gekennzeichnet, dass man

a) zur Herstellung von Verbindungen der allgemeinen Formel I, bei denen m den Wert 0 hat, eine Verbindung der Formel II

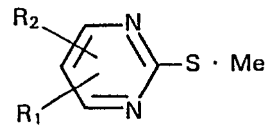

(II)

worin $R_1$ und $R_2$ die oben angegebene Bedeutung haben und Me für ein Alkaliatom steht, mit einem Picolylchlorid-Hydrohalogenid der allgemeinen Formel III

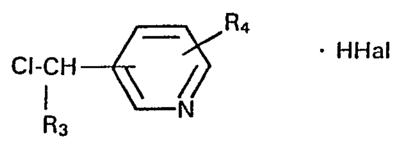

(III)

worin Hal ein Halogenatom bedeutet und in der die Chlormethylgruppierung in 2-, 3- oder 4-Stellung des Pyridinrestes steht und $R_3$ und $R_4$ die oben angegebene Bedeutung haben,
in der wässrig-alkoholischen Alkalihydroxidlösung, wobei das Alkalihydroxid im Überschuss über die stöchiometrische Menge vorliegt, umsetzt oder dass man

b) zur Herstellung von Verbindungen, bei denen m den Wert 1 hat, eine Verbindung der allgemeinen Formel IV

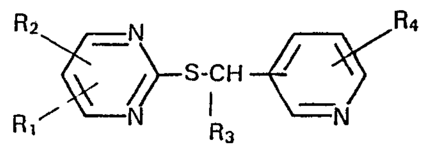

(IV)

in der $R_1$ bis $R_4$ die oben angegebene Bedeutung haben,
in einem inerten Lösungsmittel mit einem äquimolaren Anteil eines Oxidationsmittels versetzt und die erhaltene Sulfinylverbindung der allgemeinen Formel V

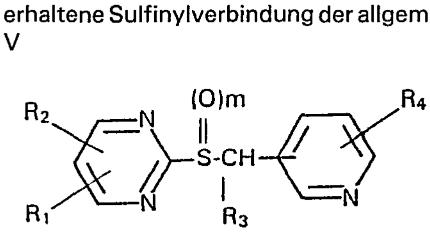

(V)

isoliert oder

c) zur Herstellung von Verbindungen, bei denen m den Wert 0 hat, ein Pyrimidin-Thioalkylpyridin-Derivat der allgemeinen Formel IV

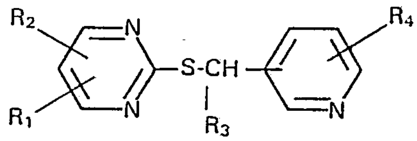

(IV)

in der $R_1$ bis $R_4$ die oben angegebenen Bedeutungen haben,
in einem Lösungsmittel mit einer äquimolaren Menge eines Alkylhalogenids zu einer Verbindung der allgemeinen Formel VI

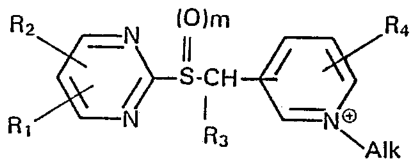

Hal$^\ominus$ (VI)

um setzt und

d) gegebenenfalls die in a) bis c) erhaltene Verbindung in ein physiologisch annehmbares Salz umwandelt.

2. Verfahren nach Anspruch 1 zur Herstellung von Pyrimidin-Thioalkylpyridin-Derivaten, bei denen m den Wert 0 hat, $R_1$ und $R_2$ unabhängig voneinander ein Wasserstoffatom, eine $C_1$-$C_4$-Alkyl-, Halogen-, Amino- oder Hydroxygruppe, die in 3-, 4- oder 5-Stellung am Pyrimidinring gebunden sein können, bedeuten, $R_3$ und $R_4$ jeweils für ein Wasserstoffatom stehen und $R_5$ ein freies Elektronenpaar bedeutet.

3. Verfahren nach Anspruch 1 zur Herstellung von Pyrimidin-Thioalkylpyridin-Derivaten, bei denen $R_3$ für eine $C_1$-$C_4$-Alkylgruppe steht und $R_4$ ein Wasserstoffatom oder eine $C_1$-$C_4$-Alkylgruppe bedeutet.

4. Verfahren nach Anspruch 1 zur Herstellung von Pyrimidin-Thioalkylpyridin-Derivaten, bei denen m den Wert 1 hat, $R_1$ und $R_2$ ein Wasserstoffatom bedeueten und $R_5$ ein freies Elektronenpaar ist.

5. Verfahren nach Anspruch 1 zur Herstellung von Pyrimidin-Thioalkylpyridin-Derivaten, bei denen m den Wert 0 hat, $R_5$ für eine $C_1$-$C_4$-Alkylgruppe steht und X ein Halogenatom bedeutet.

**Claims for the contracting states:**
BE, CH, DE, FR, GB, IT, LI, LU, NL, SE

1. Pyrimidine-thioalkylpyridine derivatives corresponding to the general formula

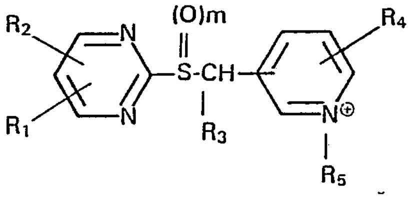

X$^\ominus$ (I)

wherein $R_1$ to $R_4$ denote, independently of one another, hydrogen, $C_1$-$C_4$ alkyl, halogen, amino or hydroxyl groups, $R_5$ denotes a free electron pair or a $C_1$ to $C_4$ alkyl group, X denotes a halogen atom when $R_5$ denotes a $C_1$-$C_4$ alkyl group, m has the value 0 or 1, and the pyrimidine-thioalkyl group is attached in the 2-, 3- or 4-position of the pyridine ring, and their therapeutically acceptable acid addition salts.

2. Pyrimidine-thioalkylpyridine derivatives according to claim 1, characterised in that m has the value 0, $R_1$ and $R_2$ denote, independently of one another, a hydrogen atom, a $C_1$-$C_4$-alkyl, halogen, amino or hydroxyl group, which groups may be attached to the pyrimidine ring in the 3-, 4- or 5-position, $R_3$ and $R_4$ represent each a hydrogen atom and $R_5$ denotes a free electron pair.

3. Pyrimidine-thioalkylpyridine derivatives according to claim 1, characterised in that $R_3$ stands for a $C_1$-$C_4$ alkyl group and $R_4$ denotes a hydrogen atom or a $C_1$-$C_4$ alkyl group.

4. Pyrimidine-thioalkylpyridine derivatives according to claim 1 or 3, characterised in that m has the value 1, $R_1$ and $R_2$ denote a hydrogen atom and $R_5$ is a free electron pair.

5. Pyrimidine-thioalkylpyridine derivatives according to one of the claims 1 to 3, characterised in that m has the value o, $R_5$ stands for a $C_1$-$C_4$ alkyl group and X denotes a halogen atom.

6. Process for the preparation of pyrimidine-thioalkylpyridine derivatives according to claim 1, characterised in that

a) for the preparation of compounds corresponding to the general formula I in which m has the value 0, a compound corresponding to the formula II

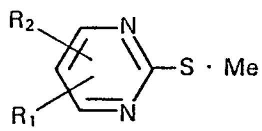

(II)

wherein $R_1$ and $R_2$ have the meaning indicated in claim 1 and Me stands for an alkali metal atom, is reacted with a picolyl chloride-hydrohalide corresponding to the general formula III

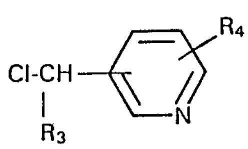

· HHal (III)

wherein Hal denotes a halogen atom and in which the chloromethyl group is in the 2-, 3- or 4-position of the pyridine group and $R_3$ and $R_4$ have the meaning indicated in claim 1
in the aqueous alcoholic alkali metal hdroxide solution in which the alkali metal hydroxide is present in excess of the stoichiometric quantity,
or in that

b) for the preparation of compounds in which m

has the value 1, a compound corresponding to the general formula IV

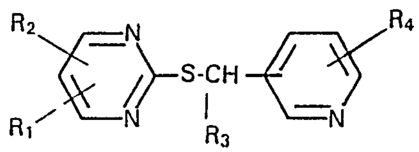
(IV)

wherein $R_1$ to $R_4$ have the meaning indicated in claim 1, has added thereto, in an inert solvent, an equimolar quantity of an oxidising agent, and the resulting sulphinyl compound corresponding to the general formula V

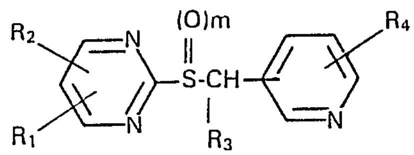
(V)

is isolated, or

c) for the preparation of compounds in which m has the value 0, a pyrimidine-thioalkylpyridine derivative corresponding to the general formula IV

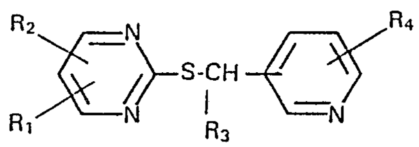
(IV)

wherein $R_1$ to $R_4$ have the meaning indicated in claim 1 is reacted in a solvent with an equimolar quantity of an alkyl halide to form a compound corresponding to the general formula VI

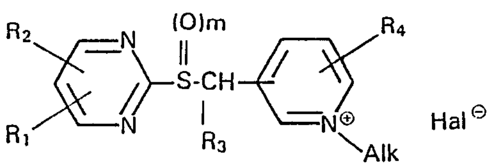
(VI)

and

d) the compound obtained in a) to c) is optionally converted into a physiologically acceptable salt.

7. Medicament, characterised in that in addition to the usual auxiliary agents and carriers it contains at least one pyrimidine-thioalkylpyridine derivative acording to claims 1 to 6.

**Claims for the contracting state:** AT

1. Process for the preparation of pyrimidine-thioalkylpyridine derivatives corresponding to the general formula I

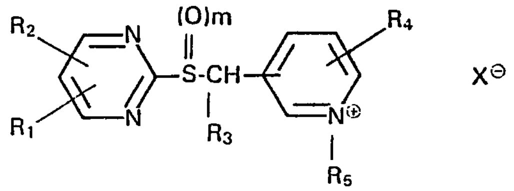
(I)

wherein $R_1$ to $R_4$ denote, independently of one another, hydrogen, $C_1$-$C_4$ alkyl, halogen, amino or hydroxyl groups, $R_5$ denotes a free electron pair or a $C_1$-$C_4$-alkyl group, X stands for a halogen atom when $R_5$ stands for a $C_1$-$C_4$ alkyl group, m has the value 0 or 1, and the pyrimidine-thioalkyl group is attached in the 2-, 3- or 4-position of the pyridine ring, and their therapeutically acceptable acid addition salts, characterised in that

a) for the preparation of compounds corresponding to the general formula I in which m has the value 0, a compound corresponding to formula II

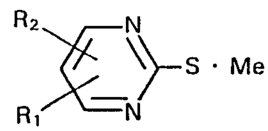
(II)

wherein $R_1$ and $R_2$ have the meaning indicated above and Me stands for an alkali metal atom is reacted with a picolyl chloride hydrohalide corresponding to the general formula III

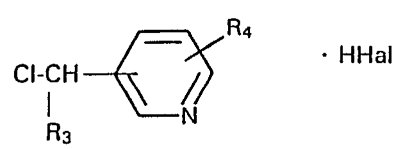
(III)

wherein Hal denotes a halogen atom and in which the chloromethyl group is in the 2,- 3- or 4-position of the pyridine group and $R_3$ and $R_4$ have the meaning indicated above, in the aqueous alcoholic alkali metal hydroxide solution, the alkali metal hydroxide being present in excess of the stoichiometric quantity,

or in that

b) for the preparation of compounds in which m has the value 1, a compound corresponding to the general formula IV

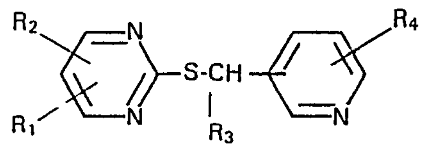
(IV)

wherein $R_1$ to $R_4$ have the meanings indicated above, has added thereto an aquimolar proportion of an oxidising agent in an inert solvent and the resulting sulphinyl compound corresponding to the general formula V

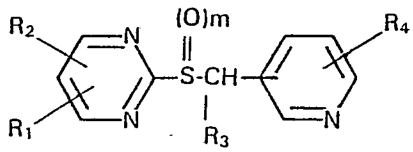

(V)

is isolated, or

c) for the preparation of compounds in which m has the value 0, a pyrimidine-thioalkylpyridine derivative corresponding to the general formula IV

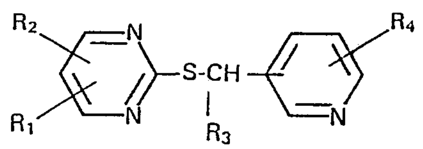

(IV)

wherein $R_1$ to $R_4$ have the meanings indicated above is reacted in a solvent with an equimolar quantity of an alkyl halide to form a compound corresponding to the general formula VI

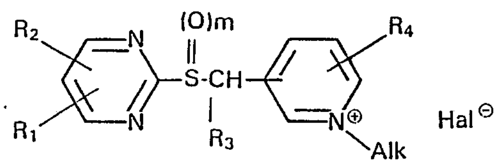

(VI)

and

d) optionally the compound obtained in a) to c) is converted into a physiologically acceptable salt.

2. Process according to claim 1 for the preparation of pyrimidine-thioalkylpyridine derivatives in which m has the value 0, $R_1$ and $R_2$ denote, independently of one another, a hydrogen atom, a $C_1$-$C_4$ alkyl, halogen, amino or hydroxyl group which may be attached to the pyrimidine ring in the 3-, 4- or 5-position, $R_3$ and $R_4$ denote each a hydrogen atom and $R_5$ denotes a free electron pair.

3. Process according to claim 1 for the preparation of pyrimidine-thioalkylpyridine derivatives in which $R_3$ stands for a $C_1$-$C_4$ alkyl group and $R_4$ denotes a hydrogen atom or a $C_1$-$C_4$ alkyl group.

4. Process according to claim 1 for the preparation of pyrimidine-thioalkypyridine derivatives in which m has the value 1, $R_1$ and $R_2$ denote a hydrogen atom and $R_5$ is a free electron pair.

5. Process according to claim 1 for the preparation of pyrimidine-thioalkylpyridine derivatives in which m has the value 0, $R_5$ stands for a $C_1$-$C_4$ alkyl group and X denotes a halogen atom.

**Revendications pour les Etats contractants:**
BE, CH, DE, FR, GB, IT, LI, LU, NL, SE

1. Dérivés de la pyrimidine-thioalkylpyridine répondant à la formule générale I:

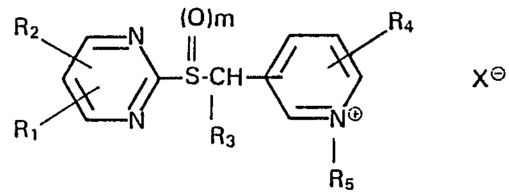

(I)

dans laquelle $R_1$ à $R_4$ représentent indépendamment les uns des autres de l'hydrogène, des groupes alkyle en $C_1$ à $C_4$, halogène, amino ou hydroxy, $R_5$ représente une paire d'électrons libres ou un groupe alkyle en $C_1$ à $C_4$, X représente, dans le cas où $R_5$ représente un groupe alkyle en $C_1$ à $C_4$, un atome d'halogène, m a pour valeur 0 ou 1, la liaison du groupe pyrimidine-thioalkyle étant réalisée en position 2, 3 ou 4 du noyau pyridique, ainsi que leurs sels d'addition d'acides thérapeutiquement compatibles.

2. Dérivés de pyrimidine-thioalkylpyridine selon la revendication 1, caractérisés en ce que m a pour valeur 0, $R_1$ et $R_2$ représentent, indépendamment l'un de l'autre, un atome d'hydrogène, un groupe alkyle en $C_1$ à $C_4$, halogène, amino ou hydroxy, pouvant être lié au noyau pyrimidique en position 3, 4 ou 5, $R_3$ et $R_4$ représentent chacun un atome d'hydrogène et $R_5$ représente une paire d'électrons libres.

3. Dérivés de pyrimidine-thioalkylpyridine selon la revendication 1, caractérisés en ce que $R_3$ représente un groupe alkyle en $C_1$ à $C_4$ et en ce que $R_4$ représente un atome d'hydrogène ou un groupe alkyle en $C_1$ à $C_4$.

4. Dérivés de pyrimidine-thioalkylpyridine selon la revendication 1 ou 3, caractérisés en ce que m a pour valeur 1, $R_1$ et $R_2$ représentent un atome d'hydrogène, et $R_5$ est une paire d'électrons libres.

5. Dérivés de pyrimidine-thioalkylpyridine selon l'une des revendications 1 à 3, caractérisés en ce que m a pour valeur 0, $R_5$ représente un groupe alkyle de $C_1$ à $C_4$ et X représente un atome d'halogène.

6. Procédé de préparation de dérivées de pyrimidine-thioalkylpyridine selon la revendication 1, caractérisé en ce que:

a) pour préparer des composés répondant à la formule générale I, dans laquelle m a pour valeur 0, on fait réagit un composé répondant à la formule II:

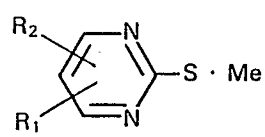

(II)

dans laquelle $R_1$ et $R_2$ ont la signification indiquée dans la revendication 1 et Me représente un atome de métal alcalin, sur un halohydrate de chlorure de picolyle répondant à la formule générale III:

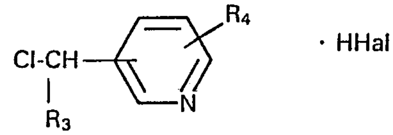

(III)

dans laquelle Hal représente un atome d'halogène et dans laquelle le groupement chlorométhyle est en position 2, 3 ou 4 du reste pyridinique et $R_3$ et $R_4$ ont la signification indiquée dans la revendication 1, dans la solution alcoolique aqueuse d'hydroxyde alcalin, l'hydroxyde alcalin étant présent en excès par rapport à la proportion stoechiométrique, ou bien

b) pour préparer des composés dans lesquels m a pour valeur 1, on fait réagir un composé répondant à la formule générale IV:

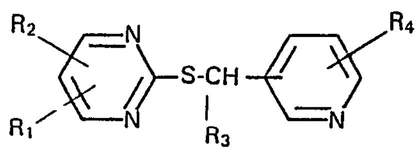

(IV)

dans laquelle $R_1$ à $R_4$ ont la signification indiquée dans la revendication 1, dans un solvant inerte, avec une proportion équimolaire d'un oxydant et l'on isole le composé sulfinylique obtenu, répondant à la formule générale V:

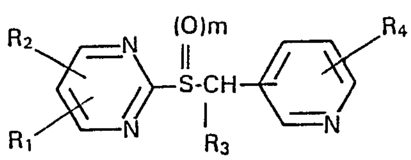

(V)

ou bien

c) pour préparer des composés dans lesquels m a la valeur 0, on fait réagir un dérivé de pyrimidine-thioalkylpyridine répondant à la formule générale IV:

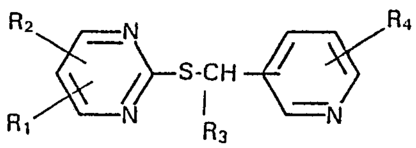

(IV)

dans laquelle $R_1$ à $R_4$ ont les significations indiquées dans la revendication 1,
dans un solvant, avec une proportion équimolaire d'un halogénure d'alkyle pour obtenir un composé répondant à la formule générale VI:

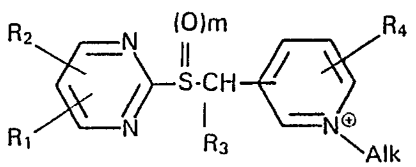

Hal $^{\ominus}$ (VI)

et

d) l'on transforme éventuellement le composé obtenu dans les stades a) à c) en un sel physiologiquement acceptable.

7. Médicament, caractérisé en ce qu'il renferme, en plus de substances auxiliaires et de supports usuels, au moins un dérivé de pyrimidine-thioalkylpyridine selon l'une des revendications 1 à 6.

**Revendications pour l'Etat contractant:** AT

1. Procédé de préparation de dérivés de pyrimidine-thioalkylpyridine répondant à la formule générale I:

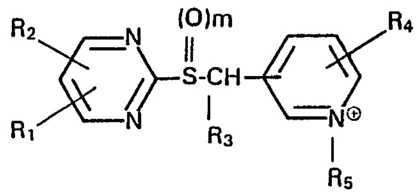

$X^{\ominus}$ (I)

dans laquelle $R_1$ à $R_4$ représentent, indépendamment les uns des autres, de l'hydrogène, des groupes alkyle en $C_1$ à $C_4$, halogène, amino ou hydroxy, $R_5$ représente une paire d'électrons libres ou un groupe alkyle en $C_1$ à $C_4$, X représente, dans le cas où $R_5$ est un groupe alkyle en $C_1$ à $C_4$, un atome d'halogène, m a pour valeur 0 ou 1, la liaison du groupe pyrimidine-thioalkyle étant réalisée en position 2, 3 ou 4 du noyau pyridique, ainsi que de leurs sels d'addition d'acides thérapeutiquement compatibles, caractérisé en ce que:

a) pour préparer des composés répondant à la formule générale I, pour lesquels m a pour valeur 0, on fait réagir un composé répondant à la formule générale II:

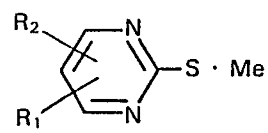

(II)

dans laquelle $R_1$ et $R_2$ ont la signification indiquée précédemment et Me représente un atome de métal alcalin, sur un halohydrate de chlorure de picolyle répondant à la formule générale III:

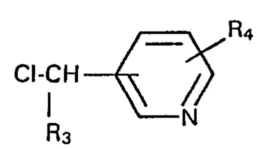

· HHal (III)

dans laquelle Hal représente un atome d'halogène et dans laquelle le groupement chlorométhyle est en position 2, 3 ou 4 du reste pyridinique et $R_3$ et $R_4$ ont la signification indiquée ci-dessus,
dans la solution alcoolique aqueuse d'hydroxyde alcalin, l'hydroxyde alcalin étant présent en excès par rapport à la proportion stoechiométrique, ou bien,

b) pour préparer des composés pour lesquels m a

pour valeur 1, on fait réagir un composé répondant à la formule générale IV:

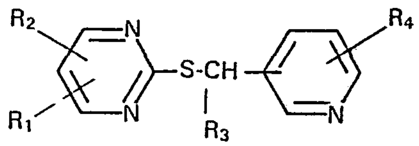

(IV)

dans laquelle $R_1$ à $R_4$ ont les significations indiquées précedemment, dans un solvant inerte, avec une proportion équimolaire d'un oxydant et l'on isole le composé sulfinylique obtenu répondant à la formule générale V:

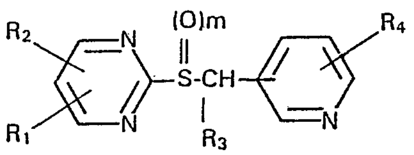

(V)

ou bien

c) pour préparer des composés pour lesquels m a pour valeur 0, on fait réagir un dérivé de pyrimidine-thioalkylpyridine répondant à la formule générale IV:

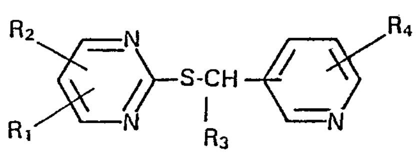

(IV)

dans laquelle $R_1$ à $R_4$ ont les significations indiquées précédemment, dans un solvant, sur une proportion équimolaire d'un halogénure d'alkyle pour obtenir un composé répondant à la formule générale VI:

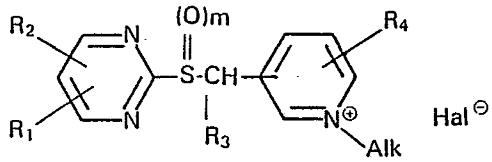

(VI)

et

d) l'on transforme éventuellement le composé obtenu au cours des stades a) à c) en un sel physiologiquement acceptable.

2. Procédé selon la revendication 1 pour préparer des dérivés de pyrimidine-thioalkylpyridine, pour lesquels m a pour valeur 0, $R_1$ et $R_2$ représentent, indépendamment l'un de l'autre, un atome d'hydrogènem un groupe alkyle en $C_1$ à $C_4$, halogène, amino ou hydroxy, pouvant être lié au noyau pyrimidique en position 3, 4 ou 5, $R_3$ et $R_4$ représentent chacun un atome d'hydrogène et $R_5$ représente une paire d'électrons libres.

3. Procédé selon la revendication 1 pour préparer des dérivés de pyrimidine-thioalkylpyridine pour lesquels $R_3$ représente un groupe alkyle en $C_1$ à $C_4$ et $R_4$ représente un atome d'hydrogène ou un groupe alkyle en $C_1$ à $C_4$.

4. Procédé selon la revendication 1 pour préparer des dérivés de pyrimidine-thioalkylpyridine, pour lesquels m a pour valeur 1, $R_1$ et $R_2$ représentent un atome d'hydrogène et $R_5$ est une paire d'électrons libres.

5. Procédé selon la revendication 1 pour préparer des dérivés de pyrimidine-thioalkylpyridine, pour lesquels m a pour valeur 0, $R_5$ représente un groupe alkyle de $C_1$ à $C_4$ et X représente un atome d'halogène.